# FASCICULE DE BREVET EUROPEEN

(11) **EP 1 593 356 B1**
(45) Date de publication et mention de la délivrance du brevet: **18.01.2012**
(21) Numéro de dépôt: 05005134.1
(22) Date de dépôt: 29.01.1999
(51) Int. Cl.: A61F 2/44

(54) **Implant pour remplacer une vertèbre**
Implantat zum Ersetzen eines Wirbelknochens
Implant for replacing a vertebra

(30) Priorité: 30.01.1998 FR 9801053
(43) Date de publication de la demande: 09.11.2005
(62) Demande divisionnaire de: 99901658.7
(73) Titulaire: Stryker Spine, 33610 Cestas (FR)
(72) Inventeur: Baccelli, Christian, 33650 Saint Medard d'Eyrans (FR); Crozet, Yves, 4512 Bellach (CH)
(74) Mandataire: Texier, Christian

(56) Documents cités:
- DE-A- 19 622 827
- DE-A1- 3 023 942
- DE-A1- 19 519 101
- FR-A- 2 730 158
- US-A- 4 554 914
- WPI WORLD PATENT INF, 23 avril 1993 (1993-04-23), XP002103980

## Description

L'invention concerne les implants destinés à remplacer une vertèbre au moins partiellement, par exemple à la suite de l'ablation de celle-ci.

On connaît d'après le document EP-0 567 424-A1 un tel implant, comprenant un corps intermédiaire et deux parties d'appui destinées à venir en appui contre les plateaux vertébraux des vertèbres adjacentes à l'espace laissé libre par la vertèbre ôtée. Chaque partie d'appui est assemblée à une extrémité du corps intermédiaire suivant une liaison vis-écrou, de sorte que la rotation de chaque partie d'appui par rapport au corps permet de faire varier la longueur totale de l'implant. Toutefois, l'assemblage des différents éléments de l'implant est relativement long à effectuer. De plus, compte tenu du nombre de pièces mobiles relativement, le réglage de la longueur de l'implant est relativement compliqué et long à effectuer, ce qui prolonge la durée de l'intervention chirurgicale. Enfin, la fabrication de l'implant nécessite la définition d'un grand nombre de surfaces de précision assurant la mobilité des pièces entre elles. Cette fabrication est longue et coûteuse.

On connaît par ailleurs du document FR-2 730 158 un implant destiné à remplacer une vertèbre, constitué de deux parties d'implant coulissant l'une dans l'autre. Les deux parties sont en contact mutuel par des dentures permettant d'accroître la longueur de l'implant par distraction des deux parties, mais interdisant de réduire cette longueur. Le réglage de la longueur de l'implant est rapide et simple à effectuer. Cependant, l'implant n'autorise pas un réglage fin de cette longueur.

On connait enfin du document US-A-4 554 914 un implant conforme au préambule de la revendication 1.

Un but de l'invention est de fournir un implant rapide à installer lors d'une intervention et permettant un réglage fin de sa longueur.

En vue de la réalisation de ce but, on prévoit selon l'invention un implant selon la revendication 1.

Ainsi, lors d'une intervention chirurgicale, on règle la dimension totale de l'implant en déplaçant seulement les deux parties de l'implant l'une par rapport à l'autre. L'opération de réglage est donc simple et rapide. De même, l'assemblage des parties de l'implant mobiles entre elles, préalablement à l'intervention ou durant celle-ci, est simple et rapide. De plus, le nombre de surfaces assurant la mobilité relative des pièces est réduit. Ces surfaces étant des surfaces de très grande précision, la fabrication de l'implant est facile et son coût est faible. La liaison vis - écrou permet un réglage fin de la longueur de l'implant. De plus, on facilite la mise en coïncidence des ouvertures, notamment lorsque les deux parties sont mobiles relativement suivant une liaison vis-écrou.

Avantageusement, au moins l'une des parties est d'un seul tenant.

Ainsi, on réduit encore le nombre de pièces à assembler.

Avantageusement, au moins l'une des parties est en plusieurs pièces.

On peut ainsi faciliter la réalisation de certaines formes de la partie concernée.

Avantageusement, au moins l'une des ouvertures est de forme allongée.

Avantageusement, l'ouverture allongée est de forme rectiligne et s'étend parallèlement à une direction de mesure de la dimension totale de l'implant.

Avantageusement, l'une des parties présente une ouverture allongée et l'autre partie présente au moins une ouverture circulaire.

Avantageusement, au moins l'une des parties présente une extrémité dentée, cette extrémité formant une extrémité de l'implant.

D'autres caractéristiques et avantages de l'invention apparaîtront encore dans la description suivante d'un mode préféré de réalisation donné à titre d'exemple non limitatif. Aux dessins annexés :
- les figures 1 et 2 sont des vues en perspective respectivement avant et après montage d'un implant selon un mode de réalisation de l'invention ;
- la figure 3 est une vue de côté d'une variante de ce mode de réalisation ;
- la figure 4 est une vue en perspective avant montage d'un implant ; et
- les figures 5 et 6 sont deux vues latérales de l'implant de la figure 4 après montage.

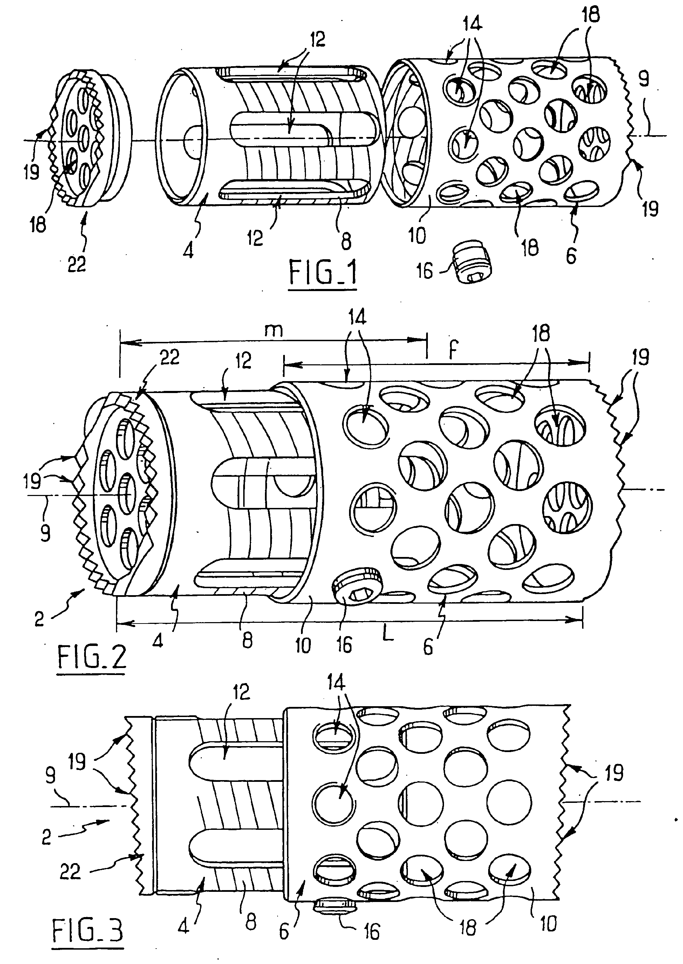

En référence aux figures 1 et 2, dans un premier mode de réalisation, l'implant 2 selon l'invention est constitué de deux parties 4, 6.

Chaque partie 4, 6 comporte un corps 8, 10 d'un seul tenant de forme tubulaire cylindrique d'axe 9. Le corps 8, ou corps mâle, est adapté à pénétrer dans le corps 10, ou corps femelle suivant une direction parallèle à l'axe 9. Le corps mâle 8 est fileté extérieurement et le corps femelle 10 est fileté intérieurement pour coopérer avec le corps mâle en réalisant une liaison vis-écrou. Une paroi latérale du corps mâle 8 présente des ouvertures ou lumières 12 de forme allongée, rectilignes, identiques entre elles, de largeur constante, parallèles entre elles et à l'axe 9, s'étendant chacune sur plus de la moitié de la longueur du corps 8 parallèlement à l'axe 9, et réparties tout autour de cet axe. Une paroi latérale du corps femelle 10 présente une série d'ouvertures de fixation ou lumières 14 identiques entre elles, de forme circulaire, s'étendant dans un même plan perpendiculaire à l'axe 9, au voisinage d'un bord proximal du corps femelle par lequel le corps mâle 8 pénètre dans le corps femelle 10. Les ouvertures circulaires 14 sont filetées. Le diamètre de ces ouvertures circulaires 14 est égal à la largeur des ouvertures allongées 12. La partie femelle 6 comporte une vis de fixation 16 adaptée à coopérer en liaison vis-écrou avec les ouvertures circulaires 14.

A un bord distal du corps femelle opposé suivant la direction axiale 9 au bord proximal, le corps femelle comporte une paroi d'extrémité présentant des ouvertures circulaires 18. Le bord distal du corps femelle présente des dents 19 s'étendant en direction opposée au bord proximal. Entre ce bord distal et les ouvertures de fixation 14, la paroi du corps femelle 10 présente d'autres ouvertures circulaires non filetées 18.

Au voisinage d'un bord distal opposé au bord proximal adapté à pénétrer dans le corps femelle, la paroi du corps mâle 8 présente un filet intérieur. La partie mâle 4 comporte un capuchon 22 comprenant une paroi cylindrique filetée pour sa fixation par liaison vis-écrou au bord distal fileté du corps mâle. Ce capuchon 22 comporte une paroi d'extrémité perpendiculaire à l'axe 9 et présentant des ouvertures circulaires 18, et des dents 19 dirigées en sens opposé au corps mâle 8. Les filets du capuchon 22 -et du bord distal du corps mâle 8 ont une longueur juste suffisante pour assurer la fixation rigide du capuchon 22 en butée axiale sur le corps mâle 8, de sorte que le capuchon peut être séparé du corps 8 par une rotation très courte autour de l'axe 9, par exemple égale à un ou deux tours. Lorsque le capuchon 22 n'est pas en butée sur le bord distal, il est relié avec jeu au corps 8. Les différentes positions du capuchon 22 par rapport au corps 8 lorsque leurs filets sont en prise ne changent pas de façon significative la longueur de la partie mâle 4 suivant l'axe 9, les filets étant très faiblement inclinés par rapport à l'axe 9. Les parties mâle et femelle ont, parallèlement à l'axe 9, des longueurs respectives invariables m et f.

Pour assembler l'implant 2, on fixe le capuchon 22 au corps 8 pour constituer la partie mâle 4. Puis, on engage la partie mâle 4 dans la partie femelle 6 en mettant en prise leurs filets respectifs. Ces filets ont chacun une longueur très importante de façon à pouvoir choisir à volonté la longueur de pénétration de la partie mâle 4 dans la partie femelle 6. Grâce à la liaison vis-écrou, la rotation relative des parties mâle et femelle permet de choisir et régler la longueur totale L de l'implant parallèlement à l'axe 9. Cette longueur L correspond à la distance séparant les deux plateaux vertébraux entre lesquels l'implant doit être installé. Lorsque la longueur L adaptée à l'espace intervertébral à occuper est obtenue, on engage la vis 16 dans l'une des ouvertures de fixation 14 du corps femelle 6 qui est en coïncidence avec une ouverture allongée 12 du corps mâle 4. Si aucune coïncidence n'est visible, il suffit alors de faire tourner les deux parties relativement sur une très faible fraction de tour pour produire cette coïncidence, et ce grâce à la forme allongée des ouvertures 12. On engage la vis 16 jusqu'à l'ouverture allongée correspondante 12, ce qui bloque toute rotation subséquente relative des deux parties. On serre enfin la vis 16 pour mettre sa tête en appui contre le corps femelle 6. Le réglage de la distance L et la fixation de la vis 16 sont effectués au moins en partie avec l'implant 2 in situ, occupant l'emplacement de la vertèbre partiellement ou totalement ôtée. Les bords distaux des parties mâle et femelle sont alors en appui contre les plateaux vertébraux respectifs des deux vertèbres adjacentes à cette dernière. Les dents 19 assurent une bonne prise de l'implant 2 sur ces plateaux et facilitent l'ostéointégration de l'implant. Toutes les ouvertures 12, 14, 18 de l'implant facilitent une ostéosynthèse pour son ostéointégration.

Dans la variante de la figure 3, les bords distaux portant les dents s'étendent dans des plans inclinés par rapport au plan perpendiculaire à l'axe 9, pour tenir compte de la configuration inclinée des plateaux vertébraux de certaines vertèbres.

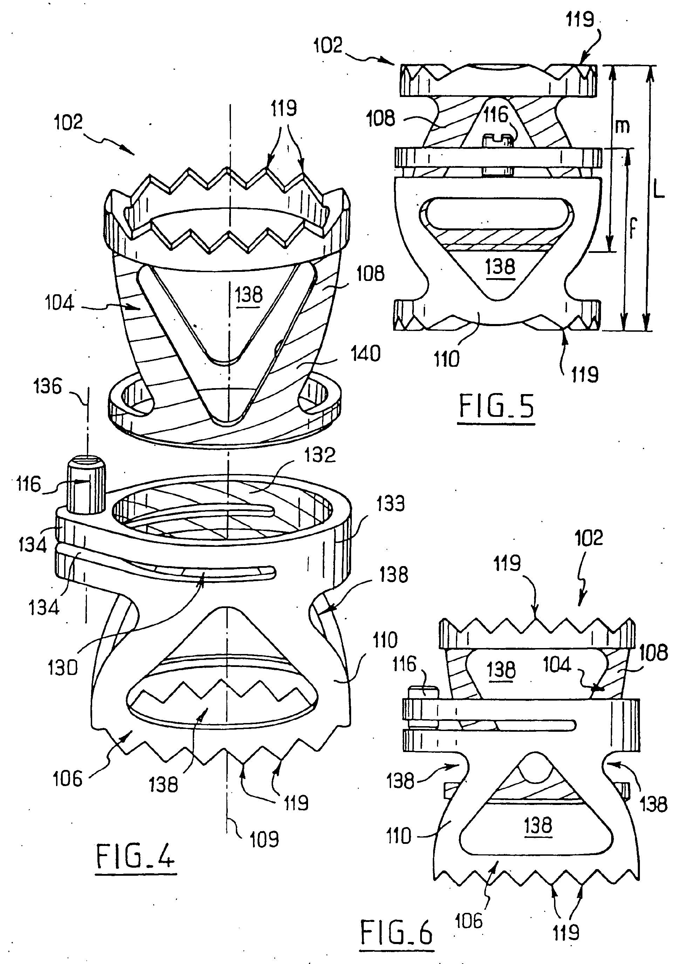

En référence aux figures 4 à 6, les références numériques des éléments correspondants ayant été augmentées de 100, les deux parties 104, 106 de l'implant réalisent comme précédemment un accouplement mâle-femelle avec une liaison vis-écrou. Cette fois, chaque bord distal et les dents qu'il porte sont d'un seul tenant avec le corps correspondant. La partie mâle 4 est d'un seul tenant. Les parties mâle 4 et femelle 6 sont dépourvues de parois d'extrémité, les extrémités de l'implant associées aux bords distaux étant ouvertes.

Le bord proximal de la partie femelle 6 présente une fente 130 s'étendant dans un plan, en l'espèce perpendiculaire à l'axe 109, sur un arc de cercle correspondant à un angle autour de l'axe supérieur à 180°, par exemple égal à 200°. Cette fente 130 délimite ainsi une bride 132 portant le bord proximal et formant un collier circulaire ininterrompu, mobile par rapport au reste du corps par déformation élastique d'une partie de jonction 133 reliant ce reste à la bride. De part et d'autre de la fente 130 et à l'opposé de la partie de jonction, la bride et le corps présentent deux lobes respectifs 134 s'étendant en saillie de la face externe du corps femelle 106, en regard l'un de l'autre. Ces lobes 134 présentent deux conduits respectifs ayant un axe commun 136, en l'espèce parallèle à l'axe 109. La partie femelle comporte une vis 116 adaptée à être engagée à partir de la bride 132 dans les deux conduits en vue de venir en prise avec un filet du conduit du corps 110, une tête de la vis étant en butée sur le lobe de la bride.

Les parois latérales des corps mâle et femelle présentent des évidements 138 de forme triangulaire s'étendant chacun de l'un à l'autre des bords proximal et distal correspondants. Sur chaque partie mâle et femelle, les évidements triangulaires 138 sont renversés en alternance autour de l'axe 109 pour définir entre eux des branches 140 reliant le bord distal au bord proximal, tous deux circulaires ininterrompus. Ces évidements très grands 138 assurent une bonne visualisation de l'implant 102 à la radiographie et favorisent son ostéointégration.

On choisit la longueur L de l'implant par rotation relative des deux parties 104, 106 comme précédemment. Lorsque la longueur L souhaitée est atteinte, on serre la vis 116 en vue de rapprocher la bride 132 du corps 106 en déformant élastiquement la partie de jonction 133. Compte tenu de la liaison filetée entre la bride 132 et le corps mâle 104, et de la liaison filetée entre les corps mâle 104 et femelle 106, ce déplacement sur une très faible course produit un coincement rigide des deux parties mâle et femelle l'une par rapport à l'autre. Alternativement, la fixation par la vis 116 pourra être prévue de sorte que ce coincement est atteint par l'éloignement de la bride 132 par rapport au corps femelle 106.

L'implant 2, 102 selon l'invention permet la mise en place d'une greffe osseuse entre deux plateaux vertébraux dans les cas où une corporectomie totale ou partielle ainsi que l'ablation des disques intervertébraux sus- et sous-jacents ont été réalisées. Une fois adapté, par le choix de sa longueur L, à la taille de l'espace à combler, l'implant 2, 102 est rempli d'os, généralement pris sur le patient. On réalise ainsi une greffe et un étaiement de la colonne.

## Revendications

1. Implant (2) pour remplacer une vertèbre au moins partiellement, l'implant étant constitué de deux parties (4,6) adaptées à être reliées mutuellement en permettant de régler une dimension totale (L) de l'implant, chaque partie ayant une dimension invariable (m, f) homoloque de la dimension totale (L) de l'implant, les parties (4,6) formant une liaison vis - écrou l'une avec l'autre **caractérisé en ce que** l'implant comporte , en outre , un élément de fixation (16) et **en ce que** chaque partie (4,6) présente au moins une ouverture latérale (12,14) les ouvertures pouvant être disposées en coïncidence pour recevoir l'élément de fixation (16).

2. Implant selon la revendication 1, **caractérisé en ce qu'**au moins l'une (6) des parties est d'un seul tenant.

3. Implant selon la revendication 1 ou 2, **caractérisé en ce qu'**au moins l'une (4) des parties est en plusieurs pièces.

4. Implant selon l'une des revendications 1 à 3 , **caractérisé en ce qu'**au moins l'une (12) des ouvertures est de forme allongée.

5. Implant selon la revendication 4 **caractérisé en ce que** l'ouverture allongée (12) est de forme rectiligne et s'étend parallèlement à une direction (9) de mesure de la dimension totale (L) de l'implant .

6. Implant selon l'une quelconque des revendications 4 ou 5 **caractérisé en ce que** l'une des parties (4) présente une ouverture allongée et l'autre partie (6) présente au moins une ouverture circulaire (14).

7. Implant selon l' une quelconque des revendications 1 à 6 **caractérisé en ce qu'**au moins l'une des parties (4, 6) présente une extrémité dentée, cette extrémité formant une extrémité de l'implant, dc l'implant (2).

## Claims

1. Implant (2) for replacing at least part of a vertebra, the implant having two parts (4, 6) adapted to be joined together and enabling a total dimension (L) of the implant to be adjusted, each part having a fixed dimension (m, f) homologous to the total dimension (L) of the implant, the parts (4, 6) forming a screw connection with each other, **characterized in that** the implant further comprises a fixing member (16) and **in that** each part (4, 6) has at least one lateral opening (12, 14) the openings being able to be superposed to receive the fixing member 16.

2. Implant according to Claim 1, **characterized in that** at least one of the parts (6) is in one piece.

3. Implant according to Claim 1 or 2, **characterized in that** at least one of the parts (4) is in more than one piece.

4. Implant according to one of Claims 1 to 3, **characterized in that** at least one of the openings (12) is elongate.

5. Implant according to Claim 4, **characterized in that** the elongate opening (12) is rectilinear and parallel to a direction (9) of measuring the total dimension (L) of the implant.

6. Implant according to either of Claims 4 and 5, **characterized in that** one part (4) has an elongate opening and the other part (6) has at least one circular opening (14).

7. Implant according to any one of Claims 1 to 6, **characterized in that** at least one of the parts (4, 6) has a toothed end forming an end of the implant (2).

## Patentansprüche

1. Implantat (2), um wenigstens teilweise einen Wirbel zu ersetzen, wobei das Implantat aus zwei Teilen (4, 6) gebildet ist, welche dafür eingerichtet sind miteinander verbunden zu werden, wodurch sie ermöglichen, eine Gesamtabmessung (L) des Implantats einzustellen, wobei jeder Teil eine unveränderbare, mit der Gesamtabmessung (L) des Implantats homologe Abmessung (m, f) aufweist, wobei die Teile (4, 6) eine Schrauben-Mutter-Verbindung miteinander bilden, **dadurch gekennzeichnet, dass** das Implantat außerdem ein Befestigungselement (16) aufweist und dass jeder Teil (4, 6) wenigstens eine Seitenöffnung (12, 14) aufweist, wobei die Öffnungen mit Deckungsgleichheit angeordnet sein können, um das Befestigungselement (16) aufzunehmen.

2. Implantat gemäß Anspruch 1, **dadurch gekennzeichnet, dass** wenigstens der eine (6) der Teile aus einem Stück ist.

3. Implantat gemäß Anspruch 1 oder 2, **dadurch gekennzeichnet, dass** zumindest der eine (4) der Teile aus mehreren Stücken besteht.

4. Implantat gemäß einem der Ansprüche 1 bis 3, **dadurch gekennzeichnet, dass** zumindest die eine (12) der Öffnungen eine längliche Form aufweist..:;

5. Implantat gemäß Anspruch 4, **dadurch gekennzeichnet, dass** die längliche Öffnung (12) eine gerade Form aufweist und sich parallel zu einer Messrichtung (9) der Gesamtabmessung (L) des Implantats erstreckt.

6. Implantat gemäß irgendeinem der Ansprüche 4 oder 5, **dadurch gekennzeichnet, dass** der eine der Teile (4) eine längliche Öffnung aufweist und der andere Teil (6) wenigstens eine kreisförmige Öffnung (14) aufweist.

7. Implantat gemäß irgendeinem der Ansprüche 1 bis 6, **dadurch gekennzeichnet, dass** wenigstens der eine Teile (4, 6) ein gezahntes Ende aufweist, wobei dieses Ende ein Ende des Implantats (2) bildet.
